# FASCICULE DE BREVET EUROPEEN

(11) **EP 0 661 370 B1**
(45) Date de publication et mention de la délivrance du brevet: **19.04.2000**
(21) Numéro de dépôt: 94402930.5
(22) Date de dépôt: 19.12.1994
(51) Int. Cl.: C10G 65/08, C10G 69/08, B01J 23/40, B01J 23/78

(54) **Catalyseur pour la réduction de la teneur en benzène dans les essences**
Katalysator zur Erniedrigung des Benzolgehaltes von Benzinen
Catalyst for lowering the benzene content in gasolines

(30) Priorité: 29.12.1993 FR 9315954
(43) Date de publication de la demande: 05.07.1995
(73) Titulaire: INSTITUT FRANCAIS DU PETROLE, 92502 Rueil-Malmaison (FR)
(72) Inventeur: Travers, Christine, F-92500 Rueil Malmaison (FR); Courty, Philippe, F-78800 Houilles (FR); Sarrazin, Patrick, F-92500 Rueil Malmaison (FR)

(56) Documents cités:
- EP-A- 0 552 070
- WO-A-92/20759
- FR-A- 1 220 868
- US-A- 3 025 248
- US-A- 3 173 856

## Description

L'invention concerne un procédé permettant la réduction de la teneur en benzène dans les fractions essences. Ces fractions essence dans le cadre de la présente invention sont de préférence généralement des mélanges de réformat léger et d'une coupe C₅-C₆ issue de la distillation directe. Ledit procédé associe l'hydrogénation d'une charge telle que le réformat léger et éventuellement l'hydrogénation d'une coupe C₅-C₆, et l'isomérisation de l'effluent issu de l'hydrogénation et éventuellement l'isomérisation de ladite coupe. Le procédé est caractérisé en ce que cette réaction d'isomérisation est réalisée sur un catalyseur particulier, comprenant du chlore et au moins un métal du groupe VIII déposé sur un support constitué d'un mélange d'alumine éta et d'alumine gamma, dans des proportions bien déterminées.

Les problèmes liés à l'environnement vont conduire conjointement à la réduction de la teneur en plomb et à la réduction de la teneur en benzène dans les fractions essences, de préférence sans diminution d'indice d'octane. Le réformage catalytique utilisé dans des conditions de forte sévérité et l'isomérisation des paraffines normales C₅-C₆ de faible indice d'octane sont les procédés les plus couramment utilisés actuellement pour obtenir des indices d'octane élevés sans adjonction de plomb. Le procédé de réformage catalytique produit des quantités importantes de benzène de haut indice d'octane. C'est pourquoi il est nécessaire de développer de nouveaux procédés permettant de réduire la teneur en benzène des essences tout en satisfaisant aux spécifications sur l'indice d'octane.

La combinaison des procédés de réformage catalytique et d'isomérisation, consistant à séparer la fraction C₅-C₆ du réformat, à l'isomériser et à l'introduire directement dans les fractions essences pour améliorer l'indice d'octane est bien connue : elle est décrite par exemple dans les brevets US-A-4.457.832, US-A-4.181.599 et US-A-3.761.392. Le traitement par isomérisation de la coupe C₅-C₆ issue de la distillation directe du pétrole brut est également bien connu. Il conduit à une amélioration considérable de l'indice d'octane de ladite coupe. La réduction de la teneur en benzène du réformat peut également être effectuée de différentes façons, telles que par exemple la modification du point de coupe du naphta entre le réformage et l'isomérisation ou la séparation du réformat en deux fractions : une fraction lourde (réformat lourd) et une fraction légère (réformat léger), tout le benzène étant concentré dans ladite fraction légère. Cette fraction légère est ensuite envoyée dans une unité d'hydrogénation qui permet de transformer le benzène en hydrocarbures cycliques, qui sont ensuite décyclisés dans une unité d'isomérisation travaillant dans des conditions sévères. Les paraffines normales ainsi formées sont isomérisées par un procédé classique d'isomérisation (US-A-5.003.118).

La demande de brevet EP-A-552070, elle, concerne un procédé comprenant l'hydrogénation de la charge caractérisée par une composition pondérale comprise dans les intervalles suivants : entre 40 et 80 % de paraffines, entre 0,5 et 7 % d'hydrocarbures cycliques et entre 6 et 45 % d'aromatiques, et caractérisée par une température maximale de distillation comprise entre 70 et 90 °C puis une isomérisation de l'effluent issu de l'hydrogénation avec mélange à ladite charge et/ou audit effluent d'une coupe C₅-C₆.

Il a été montré, dans la présente invention que, lorsque dans un procédé tel que décrit dans EP-A-552070 l'on utilise comme support du catalyseur d'isomérisation un mélange d'alumine éta et d'alumine gamma dans des proportions bien déterminées, ledit catalyseur comprenant aussi au moins un métal du groupe VIII et au moins un halogène, de préférence le chlore, on obtient de façon surprenante des performances en isomérisation améliorées et une stabilité accrue. Ledit mélange est tel que la teneur en alumine éta du support est comprise entre 85 et 95 % poids, de préférence entre 88 et 92 % poids, et de manière encore plus préférée entre 89 et 91 % poids, le complément à 100 % poids du support étant constitué d'alumine gamma.

Le procédé de la présente invention comprend donc l'hydrogénation dans une zone d'hydrogénation de la charge définie ci-après, puis l'isomérisation, dans une zone d'isomérisation, de l'effluent issu de l'hydrogénation, avec mélange à ladite charge et/ou audit effluent d'une coupe C₅-C₆, le procédé étant caractérisé en ce que l'on utilise un catalyseur particulier d'isomérisation. Le traitement conjoint en zone d'isomérisation de la charge hydrogénée et d'une coupe C₅-C₆ éventuellement hydrogénée au moins en partie conduit à l'obtention d'un effluent quasiment totalement exempt de benzène (c'est-à-dire comprenant moins de 0,1 % poids de benzène) et présentant un indice d'octane recherche supérieur ou égal à l'indice d'octane recherche du réformat léger ce qui permet d'incorporer ledit effluent directement aux fractions essences après stabilisation.

Le procédé de la présente invention est donc une amélioration du procédé décrit dans la demande de brevet EP-A-552070, et est caractérisé en ce que le catalyseur d'isomérisation comporte un support particulier qui est un mélange d'alumine éta et d'alumine gamma dans des proportions bien déterminées.

La zone d'hydrogénation et la zone d'isomérisation selon l'invention peuvent être comprises dans un même réacteur (lits superposés), ou bien dans des réacteurs séparés tels que chacune desdites zones est comprise dans au moins un réacteur. Les conditions dans lesquelles sont menées l'hydrogénation et l'isomérisation (entre autres les conditions opératoires) sont les conditions connues de l'homme du métier. Elles sont néanmoins précisées ci-après.

Les charges concernées par la présente invention sont généralement les suivantes :
charge de la zone d'hydrogénation
   une fraction légère du réformat (ou toute fraction équivalente) en mélange éventuellement avec une coupe C₅-C₆ généralement issue de la distillation directe.
Charge de la zone d'isomérisation
   l'effluent de la zone d'hydrogénation en mélange avec une coupe C₅-C₆ généralement issue de la distillation directe et éventuellement au moins partiellement traitée dans la zone d'hydrogénation comme il sera expliqué ci-dessous.

La fraction légère du réformat est obtenue par distillation dudit réformat. Elle est définie par une température maximale de distillation comprise entre 70 et 90 °C, de manière préférée entre 77 et 83 °C, et une composition pondérale par familles d'hydrocarbures comprise dans les intervalles suivants: entre 40 et 80 % de paraffines, entre 0,5 et 7 % d'hydrocarbures cycliques (tel que le cyclopentane, le méthylcyclopentane ou le cyclohexane), entre 6 et 45 % d'aromatiques. La température de distillation est généralement comprise entre la température ambiante et la température maximale de distillation (ou température de tête).

Le benzène est de façon générale essentiellement le seul composé aromatique compris dans ladite fraction.

Par ailleurs, ladite fraction peut comprendre entre 1 et 3 % d'hydrocarbures oléfiniques.

D'autre part, la fraction légère du réformat telle que décrite ci-dessus possède généralement les caractéristiques suivantes :
- le poids moléculaire moyen est compris entre 70 et 90 g/mol.,
- la masse volumique, mesurée à 15 °C, est comprise entre 0,670 et 0,780 g/cm³,
- la valeur de l'indice d'octane recherche est généralement comprise entre 75 et 90.

Tout autre charge hydrocarbonée provenant d'un autre procédé ou ensemble de procédés et définie par une composition pondérale comprise dans les intervalles suivants : entre 40 et 80 % de paraffines, entre 0,5 et 7 % d'hydrocarbures cycliques et entre 6 et 45 % d'aromatiques, et par une température maximale de distillation comprise entre 70 et 90 °C de préférence entre 77 et 83 °C, peut également être utilisée.

La composition pondérale de la coupe C₅-C₆ généralement issue de la distillation directe est variable. Elle dépend de la nature du brut à traiter dans le cas où la coupe C₅-C₆ est issue de la distillation directe.

Néanmoins, ladite coupe est définie par une teneur en paraffines généralement supérieure à 90 % poids, une teneur en hydrocarbures cycliques généralement inférieure à 10 % poids et une teneur en benzène généralement inférieure à 1,5 % poids. Son indice d'octane recherche est généralement compris entre 60 et 75.

D'autre part, ladite coupe peut contenir de très faibles teneurs de composés comportant 4 atomes de carbone par molécule (généralement moins de 0,5 % poids).

Comme indiqué précédemment on peut envoyer ensemble dans la zone d'hydrogénation d'une part une charge de type "fraction légère de réformat" et d'autre part une partie au moins de ladite coupe C₅-C₆. Dans ce cas, la teneur en coupe C₅-C₆ de la charge entrant dans la zone d'hydrogénation est alors comprise entre 10 et 90 % en poids et de manière préférée entre 15 et 55 % en poids. Mais de manière préférée, on envoie la coupe C₅-C₆ entièrement en mélange avec l'effluent issu de l'hydrogénation à l'entrée de la zone d'isomérisation, la zone d'hydrogénation étant alimentée pratiquement totalement par le réformat léger ; la teneur en coupe C₅-C₆ de la charge entrant dans la zone d'isomérisation est comprise entre 10 et 90 % en poids et de manière préférée entre 15 et 55 % en poids par rapport au mélange coupe C₅-C₆ effluent d'hydrogénation.

Il est aussi possible d'envoyer simultanément une partie de la coupe C₅-C₆ en zone d'hydrogénation avec le réformat léger et une autre partie de ladite coupe en zone d'isomérisation avec l'effluent de la zone d'hydrogénation.

Quelle que soit la charge envisagée, la pression requise pour cette étape d'hydrogénation est généralement comprise entre 1 et 60 bar absolus particulièrement entre 2 et 50 bar et de façon plus avantageuse entre 5 et 45 bar. La température opératoire de la zone d'hydrogénation est généralement comprise entre 100 et 400 °C, plus avantageusement entre 150 et 350 °C et de façon préférée entre 160 et 320 °C. La vitesse spatiale au sein de ladite zone, calculée par rapport au catalyseur, est généralement comprise entre 1 et 50 et plus particulièrement entre 1 et 30 h⁻¹ (volume de charge par volume de catalyseur et par heure). Le débit d'hydrogène au sein de ladite zone, rapporté au catalyseur, est généralement compris entre 1 et 2000 volumes (gaz aux conditions normales) par volume de catalyseur et par heure. Le rapport molaire hydrogène/hydrocarbures compris dans la charge est compris entre 0,5 et 10 et de préférence entre 1 et 3.

On utilise avantageusement la chaleur dégagée à l'étape d'hydrogénation pour préchauffer la charge de l'isomérisation.

Le catalyseur utilisé dans la zone d'hydrogénation selon le procédé de la présente invention comprend au moins un métal M choisi dans le groupe formé par le nickel, le platine et le palladium, utilisé tel quel ou de préférence déposé sur un support. Le métal M doit se trouver sous forme réduite au moins pour 50 % en poids de sa totalité. On utilise de préférence le nickel ou le platine, et de manière encore plus préférée le platine.

Lors de l'utilisation du platine ou du palladium, le catalyseur peut contenir avantageusement au moins un halogène dans une proportion en poids par rapport au catalyseur comprise entre 0,5 et 2 %. De manière préférée, on utilise le chlore ou le fluor ou la combinaison des deux dans une proportion par rapport au poids total de catalyseur comprise entre 0,5 et 1,5 %.

Dans le cas de l'utilisation du nickel, la proportion de métal M par rapport au poids total de catalyseur est comprise entre 0,1 et 60 %, plus particulièrement entre 5 et 60 % et de façon préférée entre 5 et 30 %. Dans le cas de l'utilisation du platine et/ou du palladium, la proportion totale du métal M par rapport au poids total de catalyseur est comprise entre 0,1 et 10 % et de façon préférée entre 0,05 et 5 %.

Le support est généralement choisi dans le groupe formé par l'alumine, les silice-alumines, la silice, les zéolithes, le charbon actif, les argiles et les ciments alumineux. On utilise de préférence une alumine, de surface spécifique au moins égale à 50 m²/g et de volume poreux au moins égal à 0,4 cm³/g, par exemple de surface spécifique comprise entre 50 et 350 m²/g et de volume poreux compris entre 0,4 et 1,2 cm³/g.

L'effluent issu de la zone d'hydrogénation contient généralement moins de 0,1 % poids d'aromatiques et a généralement entre 4 et 6 points d'indice d'octane de moins que la charge entrant dans ladite zone.

La zone d'isomérisation est alimentée par l'effluent de la zone d'hydrogénation comprenant le mélange réformat léger hydrogéné-coupe C₅-C₆ hydrogénée, ou par un mélange réformat léger hydrogéné-coupe C₅-C₆ non hydrogénée ou encore par un mélange réformat léger hydrogéné-coupe C₅-C₆ hydrogénée et coupe C5-C6 non hydrogénée. On ajoute généralement à la charge d'isomérisation un composé chloré, tel que le tétrachlorure de carbone ou le perchloréthylène, de façon que la teneur en chlore dans la charge soit comprise entre 50 et 5000 ppm de préférence entre 100 et 1000 ppm. L'isomérisation est généralement mise en oeuvre dans ladite zone d'isomérisation dans les conditions usuelles suivantes : la température est comprise entre 100 et 300 °C et de préférence entre 120 et 250 °C, et la pression partielle d'hydrogène est comprise entre la pression atmosphérique et 70 bar et de préférence entre 5 et 50 bar. La vitesse spatiale est comprise entre 0,2 et 10 litres et de préférence entre 0,5 et 5 litres d'hydrocarbures liquides par litre de catalyseur et par heure. Le rapport molaire hydrogène/charge à l'entrée du réacteur est tel que le rapport molaire hydrogène/charge dans l'effluent est supérieur à 0,06, de préférence compris entre 0,06 et 10.

Le catalyseur d'isomérisation utilisé selon la présente invention est caractéristique de l'invention et il comprend au moins un halogène et de préférence le chlore et au moins un métal du groupe VIII déposé sur un support constitué d'un mélange d'alumine éta et d'alumine gamma, dans des proportions bien déterminées, c'est-à-dire que ledit support est constitué d'alumine éta et d'alumine gamma, la teneur en alumine éta étant comprise entre 85 et 95 % poids par rapport au support, de préférence entre 88 et 92 % poids, et de manière encore plus préférée entre 89 et 91 % poids, le complément à 100 % poids du support étant constitué d'alumine gamma. Le métal du groupe VIII est de préférence le platine, la palladium et le nickel.

L'alumine éta utilisée dans la présente invention a une surface spécifique généralement comprise entre 400 et 600 m²/g et de manière préférée entre 420 et 550 m²/g, et un volume poreux total généralement compris entre 0,3 et 0,5 cm³/g et de manière préférée entre 0,35 et 0,45 cm³/g.

L'alumine gamma utilisée dans la présente invention possède généralement une surface spécifique comprise entre 150 et 300 m²/g et de préférence entre 180 et 250 m²/g, un volume poreux total généralement compris entre 0,4 et 0,8 cm³/g et de manière préférée entre 0,45 et 0,7 cm³/g.

Les deux types d'alumine sont mélangés et mis en forme, dans les proportions définies ci-avant, par toute technique connue de l'homme du métier, par exemple par extrusion au travers d'une filière, par pastillage ou dragéification.

D'une manière préférée, ledit support est obtenu par mélange, dans les proportions massiques précitées, d'au moins un précurseur hydraté de l'alumine éta, par exemple la bayérite, et d'au moins un précurseur hydraté de l'alumine gamma, par exemple la boehmite. Le mélange ainsi obtenu peut éventuellement être acidifié, par exemple par l'acide nitrique, puis il est mis en forme comme expliqué ci-avant.

La proportion finale d'alumine éta recherchée peut être obtenue par mélange en toute proportion d'alumine éta calcinée et d'au moins un précurseur comme ci-avant. De même la proportion finale d'alumine gamma recherchée peut être obtenue par mélange en toute proportion d'alumine gamma calcinée et d'au moins un précurseur comme ci-avant (en respectant toutefois les proportions finales indiquées plus haut entre alumine gamma et alumine éta).

Le support ainsi obtenu a une surface spécifique généralement comprise entre 300 et 550 m²/g et de préférence entre 350 et 500 m²/g et un volume poreux généralement compris entre 0,3 et 0,6 cm³/g et de préférence entre 0,35 et 0,5 cm³/g.

Au moins un métal hydrogénant du groupe VIII, de préférence choisi dans le groupe formé par le platine, le palladium et le nickel, est ensuite déposé sur ce support, par toute technique connue de l'homme du métier, par exemple par échange anionique sous forme d'acide hexachloroplatinique dans le cas du platine ou sous forme de chlorure de palladium dans le cas du palladium.

Dans le cas du platine ou du palladium, la teneur en poids est comprise entre 0,05 et 1 % et de manière préférée entre 0,1 et 0,6 %. Dans le cas du nickel la teneur pondérale est comprise entre 0,1 et 10 % et de manière préférée entre 0,2 et 5 %.

Le catalyseur ainsi préparé peut être réduit sous hydrogène puis est soumis à un traitement d'halogénation et notamment de chloration par tout composé halogéné, plus particulièrement chloré connu de l'homme du métier tel que par exemple le tétrachlorure de carbone ou le perchloréthylène. La teneur en chlore du catalyseur final est comprise de préférence entre 5 et 15 % poids et de manière préférée entre 6 et 11 % poids. Ce traitement de chloration du catalyseur peut être effectué soit directement dans l'unité avant injection de la charge ("in-situ" ) soit hors site.

Il est aussi possible de procéder au traitement de chloration préalablement au traitement de réduction du catalyseur sous hydrogène.

L'effluent obtenu à la sortie de la zone d'isomérisation présente un indice d'octane suffisamment élevé pour être incorporé aux fractions essences après stabilisation et est pratiquement totalement exempt de benzène (teneur maximale en benzène généralement égale à 0,1 % poids).

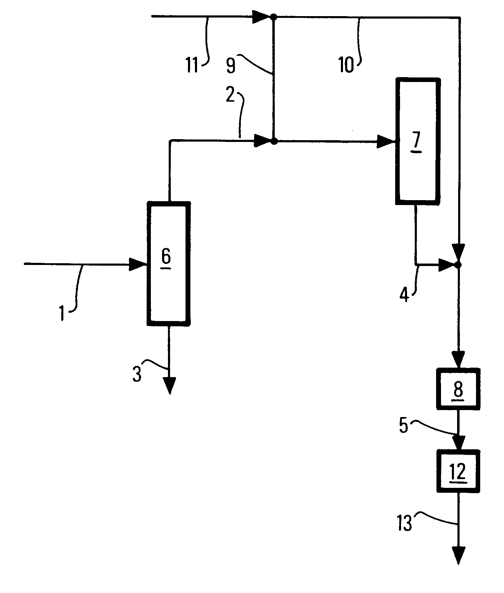

La figure unique présente un arrangement du procédé selon l'invention, dans lequel l'hydrogénation et l'isomérisation sont effectuées dans deux réacteurs (ou unités) séparé(e)s.

Un réformat stabilisé (1) est envoyé à une colonne de distillation (6), dont on sort en fond un réformat lourd (3) qui peut être utilisé directement dans les fractions essences et en tête un réformat léger (2). Ce dernier est envoyé vers une unité d'hydrogénation (7), après mélange avec une partie (9) d'une coupe C₅-C₆ issue de la distillation directe introduite par (11). On ajoute au moins un composé chloré comme ci-avant défini à l'effluent (4), obtenu après mélange entre l'effluent d'hydrogénation et l'autre partie (10) de la coupe C₅-C₆. Ledit mélange chloré est traité dans une unité d'isomérisation (8) donnant le produit final (5) qui, après stabilisation (12), peut être incorporé dans les fractions essences, par le conduit (13).

Les exemples qui suivent précisent l'invention sans en limiter la portée. Les réactions d'hydrogénation et d'isomérisation sont effectuées dans lesdits exemples dans deux réacteurs (ou unités) séparé(e)s.

### EXEMPLE 1 (selon l'invention)

Le réformat léger obtenu après distillation à 80 °C, contenant 21,5 % de benzène et présentant un indice d'octane de 80,3 est mélangé à raison de 50 % poids avec une coupe C₅-C₆ de distillation directe contenant 0,7 % de benzène et présentant un indice d'octane de 65. Les compositions de ces deux produits figurent dans le tableau 1. Le réformat léger comprend 21,5 % d'aromatiques, 4 % d'hydrocarbures cycliques et 74,5 % de paraffines. La coupe C₅-C₆ comprend 0,7 % de benzène, 7,25 % d'hydrocarbures cycliques et 92,05 % de paraffines. La charge issue du mélange, dont la composition figure également dans le tableau 1, est envoyée dans une unité d'hydrogénation à une température de 110 °C, et une pression de 40 bar. Le rapport molaire hydrogène/hydrocarbures contenus dans la charge est égal à 0,85 et la vitesse spatiale liquide est égale à 4 h⁻¹. Le catalyseur utilisé dans la section d'hydrogénation est constitué de 15 % de Ni déposé sur alumine.

L'effluent issu de l'unité d'hydrogénation, dont la composition détaillée figure tableau 1, ne contient plus de benzène mais présente un indice d'octane de 70,9. Il est alors envoyé, après ajout de 500 ppm de CCl₄, à une unité d'isomérisation fonctionnant à une température de 170° C, une pression de 30 bar et une vitesse spatiale de 2 litres d'hydrocarbures liquides par litre de catalyseur et par heure. Le rapport molaire H₂/charge à l'entrée est tel que ce même rapport soit égal à 0,07 dans l'effluent. Le catalyseur utilisé dans l'unité d'isomérisation est composé de 0,3 % poids de Pt déposé sur un support constitué de 90 % poids d'alumine éta et de 10 % poids d'alumine gamma. Le catalyseur ainsi défini est ensuite chloré à raison de 9 % poids de Cl. L'effluent sorti de l'unité d'isomérisation a la composition donnée au tableau 1. Il ne contient pratiquement plus de benzène et présente un indice d'octane de 81,5. Il est donc directement incorporable dans les fractions essences après stabilisation.

**Tableau 1**

| | **Réformat** | **Coupe C**_{**5**}**-C**_{**6**} **de distillation** | **Charge de l'hydrogénation** | **Effluent de l'hydrogénation** | **Effluent de l'isomérisation** |
|---|---|---|---|---|---|
| Légers | 6,5 | 1,0 | 3,7 | 3,7 | 6,0 |
| iC₅ | 9,9 | 18,9 | 14,4 | 14,4 | 23,7 |
| nC₅ | 7,1 | 25,4 | 16,25 | 16,25 | 7,4 |
| 22DMC₄ | 3,0 | 0,4 | 1,7 | 1,7 | 13,2 |
| 23DMC₄ | 4,1 | 1,85 | 3,0 | 3,0 | 4,9 |
| 2MC₅ | 15,8 | 11,1 | 13,45 | 13,45 | 16,5 |
| 3MC₅ | 12,5 | 9,4 | 11,0 | 11,0 | 10,1 |
| nC₆ | 12,1 | 19,6 | 15,9 | 15,9 | 6,7 |
| C₇ | 3,5 | 4,4 | 3,9 | 3,9 | 3,0 |
| CC₅ | 0,4 | 1,4 | 0,9 | 0,9 | 0,5 |
| MCC₅ | 3,6 | 4,1 | 3,85 | 3,85 | 4,9 |
| CC₆ | 0 | 1,75 | 0,85 | 11,95 | 3,1 |
| Benzène | 21,5 | 0,7 | 11,1 | - | - |
| R.O.N. | 80,3 | 65 | 72,9 | 70,9 | 81,5 |

### EXEMPLE 2 (selon l'invention)

On prépare six catalyseurs référencés de A à F, composés de 0,3 % de Pt déposé sur un support comprenant un mélange d'alumine éta et d'alumine gamma, la teneur en alumine éta variant de 85 à 95 % dans ce support comme indiqué tableau 2. Les catalyseurs ainsi définis sont chlorés à raison de 9 % poids de chlore. L'effluent en sorti de l'unité d'hydrogénation dont la composition figure tableau 1 est envoyé à une unité d'isomérisation fonctionnant dans les conditions décrites dans l'exemple 1. Les RON obtenus après isomérisation sont donnés tableau 2. On constate que le maximum de RON est obtenu pour une teneur en alumine éta dans le support comprise entre 89 et 91 %.

**Tableau 2**

| | **A** | **B** | **C** | **D** | **E** | **F** |
|---|---|---|---|---|---|---|
| Teneur en Al₂O₃ η dans le support (%) | 85 | 88 | 89 | 91 | 92 | 95 |
| RON après isomérisation | 81 | 81,2 | 81,5 | 81,5 | 81,2 | 81 |

### EXEMPLE 3 (selon l'invention)

Le réformat léger obtenu après distillation à 80 °C contenant 21,5 % de benzène, présentant un indice d'octane de 80,3 et dont la composition détaillée est donnée tableau 3, comprend 21,5 % d'aromatiques, 4 % d'hydrocarbures cycliques et 74,5 % de paraffines ; il est envoyé dans une unité d'hydrogénation à une température de 110 °C et une pression de 40 bar.
Le rapport molaire hydrogène sur hydrocarbures contenus dans la charge est égal à 0,85 et la vitesse spatiale liquide est égale à 4 h⁻¹. Le catalyseur utilisé dans la section d'hydrogénation est constitué de 15 % de Ni déposé sur alumine.
L'effluent issu de l'unité d'hydrogénation, dont la composition détaillée figure tableau 3, ne contient plus de benzène mais présente un indice d'octane de 76,5. Il est alors mélangé à raison de 50 % poids avec une coupe C₅-C₆ de distillation directe contenant 0,7 % de benzène, 92,05 % de paraffines et 7,25 % d'hydrocarbures cycliques, et présentant un indice d'octane de 65. La composition de cette coupe ainsi que la composition du mélange qui constitue la charge de l'unité d'isomérisation sont données dans le tableau 3.

L'unité d'isomérisation fonctionne dans les mêmes conditions que celles décrites dans l'exemple 1 et avec un catalyseur identique à celui décrit dans l'exemple 1.

L'effluent sorti de l'unité d'isomérisation a la composition donnée dans le tableau 3 ; il ne contient plus de benzène et présente un indice d'octane de 81,5. Cet effluent est directement incorporable dans les fractions essences après stabilisation.

**Tableau 3**

| | **Réformat** | **Coupe C**_{**5**}**-C**_{**6**} **de distillation** | **Effluent de l'hydrogénation** | **Charge de l'isomérisation** | **Effluent de l'isomérisation** |
|---|---|---|---|---|---|
| Légers | 6,5 | 1,0 | 6,5 | 3,7 | 6,0 |
| iC₅ | 9,9 | 18,9 | 9,9 | 14,4 | 23,7 |
| nC₅ | 7,1 | 25,4 | 7,1 | 16,25 | 7,4 |
| 22DMC₄ | 3,0 | 0,4 | 3,0 | 1,7 | 13,2 |
| 23DMC₄ | 4,1 | 1,85 | 4,1 | 3,0 | 4,9 |
| 2MC₅ | 15,8 | 11,1 | 15,8 | 13,45 | 16,5 |
| 3MC₅ | 12,5 | 9,4 | 12,5 | 11,0 | 10,1 |
| nC₆ | 12,1 | 19,6 | 12,1 | 15,9 | 6,7 |
| C₇ | 3,5 | 4,4 | 3,5 | 3,9 | 3,0 |
| CC₅ | 0,4 | 1,4 | 0,4 | 0,9 | 0,5 |
| MCC₅ | 3,6 | 4,1 | 3,6 | 3,85 | 4,9 |
| CC₆ | 0 | 1,75 | 21,5 | 11,60 | 3,1 |
| Benzène | 21,5 | 0,7 | - | ,35 | - |
| R.O.N. | 80,3 | 65 | 76,5 | 71,2 | 81,5 |

### EXEMPLE 4 (selon l'invention)

Le présent exemple diffère de l'exemple 1 uniquement en ce que la teneur en chlore du catalyseur utilisé dans l'unité d'isomérisation est égale à 7 % poids.

L'effluent sorti de l'unité d'isomérisation a la composition donnée au tableau 4. Il ne contient pratiquement plus de benzène et présente un indice d'octane de 80,3. Il est donc directement incorporable dans les fractions essence après stabilisation.

**Tableau 4**

| | **Réformat** | **Coupe C**_{**5**}**-C**_{**6**} **de distillation** | **Charge de l'hydrogénation** | **Effluent de l'hydrogénation** | **Effluent de l'isomérisation** |
|---|---|---|---|---|---|
| Légers | 6,5 | 1,0 | 3,7 | 3,7 | 6,0 |
| iC₅ | 9,9 | 18,9 | 14,4 | 14,4 | 22,2 |
| nC₅ | 7,1 | 25,4 | 16,25 | 16,25 | 8,9 |
| 22DMC₄ | 3,0 | 0,4 | 1,7 | 1,7 | 12,7 |
| 23DMC₄ | 4,1 | 1,85 | 3,0 | 3,0 | 4,5 |
| 2MC₅ | 15,8 | 11,1 | 13,45 | 13,45 | 16,5 |
| 3MC₅ | 12,5 | 9,4 | 11,0 | 11,0 | 10,1 |
| nC₆ | 12,1 | 19,6 | 15,9 | 15,9 | 7,6 |
| C₇ | 3,5 | 4,4 | 3,9 | 3,9 | 3,0 |
| CC₅ | 0,4 | 1,4 | 0,9 | 0,9 | 0,5 |
| MCC₅ | 3,6 | 4,1 | 3,85 | 3,85 | 4,9 |
| CC₆ | 0 | 1,75 | 0,85 | 11,95 | 3,1 |
| Benzène | 21,5 | 0,7 | 11,1 | - | - |
| R.O.N. | 80,3 | 65 | 72,9 | 70,9 | 80,3 |

### EXEMPLE 5 (comparatif)

L'exemple 5 diffère de l'exemple 4 uniquement en ce que le catalyseur utilisé dans la zone d'isomérisation (toujours composé de 0,3 % poids de Pt) a un support constitué de 50 % poids d'alumine éta et de 50 % poids d'alumine gamma.

Le catalyseur ainsi défini est ensuite soumis à un traitement de chloration. La teneur finale en chlore est de 7 % poids.

Le tableau 5 donne la composition de l'effluent sorti de l'unité d'isomérisation.

**Tableau 5**

| | **Réformat** | **Coupe C**_{**5**}**-C**_{**6**} **de distillation** | **Charge de l'hydrogénation** | **Effluent de l'hydrogénation** | **Effluent de l'isomérisation** |
|---|---|---|---|---|---|
| Légers | 6,5 | 1,0 | 3,7 | 3,7 | 5,5 |
| iC₅ | 9,9 | 18,9 | 14,4 | 14,4 | 18,4 |
| nC₅ | 7,1 | 25,4 | 16,25 | 16,25 | 12,3 |
| 22DMC₄ | 3,0 | 0,4 | 1,7 | 1,7 | 10,3 |
| 23DMC₄ | 4,1 | 1,85 | 3,0 | 3,0 | 3,9 |
| 2MC₅ | 15,8 | 11,1 | 13,45 | 13,45 | 12,9 |
| 3MC₅ | 12,5 | 9,4 | 11,0 | 11,0 | 7,9 |
| nC₆ | 12,1 | 19,6 | 15,9 | 15,9 | 15,0 |
| C₇ | 3,5 | 4,4 | 3,9 | 3,9 | 3 |
| CC₅ | 0,4 | 1,4 | 0,9 | 0,9 | 0,5 |
| MCC₅ | 3,6 | 4,1 | 3,85 | 3,85 | 5,2 |
| CC₆ | 0 | 1,75 | 0,85 | 11,95 | 5,1 |
| Benzène | 21,5 | 0,7 | 11,1 | - | - |
| R.O.N. | 80,3 | 65 | 72,9 | 70,9 | 74,8 |

Le gain d'indice d'octane (RON) entre la charge de l'isomérisation, c'est-à-dire l'effluent de l'hydrogénation, et l'effluent de l'isomérisation est très faible.

Il y a perte d'indice d'octane (RON) par rapport au réformat léger.

Note : dans les tableaux ci-dessus :
- 22DMC₄: = 2,2-diméthylbutane
- 23DMC₄: = 2,3-diméthylbutane
- 2MC₅: = 2-méthylpentane
- 3MC₅: = 3-méthylpentane
- CC₅: = cyclopentane
- MCC₅: = méthyl cyclopentane
- CC₆: = cyclohexane

## Revendications

1. Procédé de réduction de la teneur en benzène dans les fractions essences dans lequel on effectue, dans une zone d'hydrogénation, une hydrogénation d'au moins une charge d'hydrogénation définie par une composition pondérale comprise dans les intervalles suivants : entre 40 et 80 % de paraffines, entre 0,5 et 7 % d'hydrocarbures cycliques et entre 6 et 45 % d'aromatiques, et par une température maximale de distillation comprise entre 70 et 90 °C, ladite charge d'hydrogénation étant traitée en mélange ou non avec au moins une partie d'une coupe C₅-C₆ définie plus bas, puis une isomérisation, dans une zone d'isomérisation, d'une charge d'isomérisation renfermant l'effluent issu de l'hydrogénation, en mélange avec une coupe C₅-C₆ dont la teneur en paraffines est supérieure à 90 % poids, la teneur en hydrocarbures cycliques est inférieure à 10 % poids et la teneur en benzène est inférieure à 1,5 % poids, cette dite coupe C₅-C₆ pouvant avoir été au moins partiellement traitée dans la zone d'hydrogénation qui précède (en mélange avec ladite charge), le procédé étant caractérisé en ce que l'on utilise au cours de la réaction d'isomérisation un catalyseur d'isomérisation comprenant au moins un halogène et au moins un métal du groupe VIII sur un support qui est sensiblement constitué d'un mélange d'alumine éta et d'alumine gamma, la teneur en alumine éta étant comprise entre 85 et 95 % poids par rapport au support, le complément à 100 % poids du support étant de l'alumine gamma.

2. Procédé selon la revendication 1 dans lequel la teneur en alumine éta du support du catalyseur d'isomérisation est comprise entre 88 et 92 % poids.

3. Procédé selon la revendication 2 dans lequel la teneur en alumine éta du support d'isomérisation est comprise entre 89 et 91 % poids.

4. Procédé selon l'une des revendications 1 à 3 dans lequel la surface spécifique de l'alumine éta est comprise entre 400 et 600 m²/g et son volume poreux entre 0,3 et 0,5 cm³/g, l'alumine gamma ayant une surface spécifique comprise entre 150 et 300 m²/g et un volume poreux entre 0,4 et 0,8 cm³/g.

5. Procédé selon l'une des revendications 1 à 4 dans lequel la teneur en chlore du catalyseur d'isomérisation est comprise entre 5 et 15 % poids.

6. Procédé selon l'une des revendications 1 à 5 dans lequel le métal du groupe VIII compris dans le catalyseur d'isomérisation est choisi dans le groupe formé par le platine, le palladium et le nickel.

7. Procédé selon l'une des revendications 1 à 6 dans lequel ladite charge d'isomérisation comprend entre 10 et 90 % en poids de ladite coupe.

8. Procédé selon l'une des revendications 1 à 7 dans lequel la coupe C₅-C₆ est mélangée entièrement à l'effluent issu de l'hydrogénation.

9. Procédé selon l'une des revendications 1 à 8 dans lequel le catalyseur d'hydrogénation comprend au moins un métal choisi dans le groupe formé par le nickel, le platine et le palladium.

10. Procédé selon l'une des revendications 1 à 9 dans lequel la coupe C₅-C₆ est une coupe de distillation directe.

## Claims

1. A process for reducing the benzene content in petrol fractions in which at least one hydrogenation feed defined with the following composition by weight : 40 % to 80 % of paraffins, 0.5 % to 7 % of cyclic hydrocarbons and 6 % to 45 % of aromatics, and with a maximum distillation temperature of 70°C to 90°C is hydrogenated in a hydrogenation zone, said hydrogenation feed being mixed or not with at least a portion of a C₅-C₆ cut which will be defined below, followed by isomerisation, in an isomerisation zone, of an isomerisation feed containing the effluent from the hydrogenation step, mixed with a C₅-C₆ cut with a paraffin content of more than 90 % by weight, a cyclic hydrocarbon content of less than 10 % by weight and a benzene content of less than 1.5 % by weight, said C₅-C₆ cut optionally having been treated at least in part in the preceding hydrogenation zone (mixed with said feed), the process being characterised in that an isomerisation catalyst is used during the isomerisation reaction which contains at least one halogen and at least one metal from group VIII on a support which is substantially constituted by a mixture of eta alumina and gamma alumina, the eta alumina content being between 85 % and 95 % by weight with respect to the support, and the complement to 100 % of the support being gamma alumina.

2. A process according to claim 1, wherein the eta alumina content of the isomerisation catalyst is between 88 % and 92 % by weight.

3. A process according to claim 2 wherein the eta alumina content of the support is between 89 % and 91 % by weight.

4. A process according to any one of claims 1 to 3, wherein the specific surface area of the eta alumina is between 400 and 600 m²/g and the pore volume is between 0.3 and 0.5 cc/g, the gamma alumina having a specific surface area between 150 and 300 m²/g and a pore volume between 0.4 and 0.8 cc/g.

5. A process according to any one of claims 1 to 4, wherein the chlorine content of the isomerisation catalyst is between 5 % and 15 % by weight.

6. A process according to any one of claims 1 to 5, wherein the group VIII metal in the isomerisation catalyst is selected from the group formed by platinum, palladium and nickel.

7. A process according to any one of claims 1 to 6, wherein said isomerisation feed contains 10 % to 90 % of said cut.

8. A process according to any one of claims 1 to 7, wherein the C₅-C₆ cut is completely mixed with the hydrogenation effluent.

9. A process according to any one of claims 1 to 8, wherein the hydrogenation catalyst contains at least one metal selected from the group formed by nickel, platinum and palladium.

10. A process according to any one of claims 1 to 9, wherein the C₅-C₆ cut is a straight run cut.

## Patentansprüche

1. Verfahren zur Verminderung des Benzolgehaltes in Benzinfraktionen, bei dem man in einer Hydrierzone eine Hydrierung wenigstens einer Hydriercharge vornimmt, die definiert ist durch eine Gewichtszusammensetzung in den folgenden Intervallen: zwischen 40 und 80 % Paraffine, zwischen 0,5 und 7 % cyclische Kohlenwasserstoffe und zwischen 6 und 45 % Aromate und bei einer maximalen Destillationstemperatur zwischen 70 und 90°C, wobei diese Hydriercharge, gegebenenfalls im Gemisch, mit wenigstens einem Teil einer weiter unten definierten C₅-C₆-Fraktion behandelt wird, dann einer Isomerisierung in einer Isomerisierungszone einer Isomerisierungscharge vorgenommen wird, welche den aus der Hydrierung stammenden Abstrom umschließt, im Gemisch mit einer C₅-C₆-Fraktion, deren Gehalt an Paraffinen größer als 90 Gew.-% ist, wobei der Gehalt an cyclischen Kohlenwasserstoffen unter 10 Gew.-% und der Benzolgehalt unter 1,5 Gew.-% liegt, wobei diese C₅-C₆-Fraktion wenigstens teilweise in der vorhergehenden Hydrierzone (im Gemisch mit dieser Charge) behandelt sein kann, dadurch gekennzeichnet, daß man während der Isomerisierungsreaktion einen Isomerisierungskatalysator verwendet, der wenigstens ein Halogen und wenigstens ein Metall der Gruppe VIII auf einem Träger umfaßt, der im wesentlichen gebildet ist aus einem Gemisch aus Aluminiumoxid und Gamma-Aluminiumoxid, wobei der Gehalt an Eta-Aluminiumoxid zwischen 85 und 95 Gew.-%, bezogen auf den Träger, liegt, und das Komplement auf 100 Gew.-% des Trägers Gamma-Aluminiumoxid ist.

2. Verfahren nach Anspruch 1, bei dem der Gehalt an Eta-Aluminiumoxid des Trägers des Isomerisierungskatalysators zwischen 88 und 92 Gew.-% liegt.

3. Verfahren nach Anspruch 2, bei dem der Gehalt an Eta-Aluminiumoxid des Isomerisierungsträgers zwischen 89 und 91 Gew.-% liegt.

4. Verfahren nach einem der Ansprüche 1 bis 3, bei dem die spezifische Oberfläche des Eta-Aluminiumoxids zwischen 400 und 600 m²/g und sein Porenvolumen zwischen 0,3 und 0,5 cm³/g liegt, wobei das Gamma-Aluminiumoxid eine spezifische Oberfläche zwischen 150 und 300 m²/g und ein Porenvolumen zwischen 0,4 und 0,8 cm³/g hat.

5. Verfahren nach einem der Ansprüche 1 bis 4, bei dem der Gehalt an Chlor des Isomerisierungskatalysators zwischen 5 und 15 Gew.-% liegt.

6. Verfahren nach einem der Ansprüche 1 bis 5, bei dem das Metall der Gruppe VIII, das im Isomerisierungskatalysator enthalten ist, gewählt ist aus der Gruppe, die durch Platin, Palladium und Nickel gebildet ist.

7. Verfahren nach einem der Ansprüche 1 bis 6, bei dem diese Isomerisierungscharge zwischen 10 und 90 Gew.-% dieser Fraktion umfaßt.

8. Verfahren nach einem der Ansprüche 1 bis 7, bei dem die C₅-C₆-Fraktion völlig mit dem aus der Hydrierung stammenden Abstrom vermischt wird.

9. Verfahren nach einem der Ansprüche 1 bis 8, bei dem der Hydrierungskatalysator wenigstens ein Metall umfaßt, das aus der durch Nickel, Platin und Palladium gewählten Gruppe gebildet ist.

10. Verfahren nach einem der Ansprüche 1 bis 9, bei dem die C₅-C₆-Fraktion eine Fraktion der direkten Destillation ist.
